# EUROPEAN PATENT SPECIFICATION

(11) **EP 0 940 146 B1**
(45) Date of publication and mention of the grant of the patent: **04.12.2002**
(21) Application number: 99300240.1
(22) Date of filing: 13.01.1999
(51) Int. Cl.: A61L 2/26

(54) **Autoclaves**
Autoklaven
Autoclaves

(30) Priority: 05.02.1998 GB 9802344
(43) Date of publication of application: 08.09.1999
(73) Proprietor: Eschmann Holdings Limited, Lancing, West Sussex BN15 8TJ (GB)
(72) Inventor: Clayton, Patrick Brian, Worthing, West Sussex BN11 5HT (GB)
(74) Representative: Jenkins, Peter David

(56) References cited:
- GB-A- 1 039 499
- GB-A- 1 088 953
- GB-A- 1 555 295
- US-A- 4 203 947
- US-A- 5 641 085

## Description

This invention relates to autoclaves of the kind having a chamber, a door closing an opening to the chamber and an annular seal extending around the chamber between the chamber and the door.

Autoclaves are used to treat articles with steam at elevated pressure and temperature, such as to effect sterilization. The autoclave chamber is closed by a door through which articles are placed in and removed from the chamber. The door seal usually comprises a flexible gasket bearing on the inside of the door and on the outside of a flange formed around the chamber. There is a tendency for water to collect on this seal and to spill out when the door is opened.

GB-A-1088953 discloses an autoclave having a hinged door which is adapted to provide a pressure-light seal against the open end of the sterilisation compartment of the autoclave. A water-heating compartment is disposed below the sterilisation compartment and connected to the latter by a pipe.

US-A-4203947 discloses a steam sterilising apparatus with a double-walled chamber structure which includes an inner, open-ended vessel defining, with a door, a sterilising chamber. The door can be firmly locked to tightly seal the chamber during the sterilising operation. A drain pipe connects the vessel to a heat exchanger.

GB-A-1039499 discloses an autoclave having the features of the pre-characterising portion of claim 1.

It is an object of the present invention to provide an improved autoclave.

According to the present invention there is provided an autoclave having a chamber, a door closing an opening to the chamber and an annular seal, extending around a flange of the opening of the chamber, between the chamber and the door, characterised in that the flange has a passage opening just above the lower part of the annular seal, and the autoclave includes a valve that prevents flow of liquid through the passage from the chamber during a treatment cycle and allows liquid collecting above the seal to flow away before opening the door after a treatment cycle.

The seal preferably has an outer and inner resilient rib, the outer rib bearing against an inner surface of the door and the inner rib bearing against an outer surface of the chamber. The two ribs preferably diverge away from one another, forming a V shape in section, the outer rib being longer than the inner rib. The outer rib at the lower part of the seal preferably projects above the lower edge of the opening of the passage. The seal may be of a rubber. The valve is preferably a solenoid valve. The passage may connect with a reservoir by which liquid is supplied to the chamber before a treatment cycle. The flange preferably has a forwardly-projecting lip with the seal being located on the lip.

An autoclave and its method of operation, in accordance with the present invention, will now be described, by way of example only, with reference to the accompanying drawings, in which:
- Figure 1: is a simplified sectional side elevation of the autoclave; and
- Figure 2: is an enlarged sectional side elevation showing the lower part of the door and seal.

The autoclave has a pressure chamber 1, which is closed at its rear end 2 and is open at its forward end 3 wherein it has an outwardly-extending, radial flange 4 with a forwardly-projecting annular lip 14. The autoclave includes a conventional heating element 5 controlled by a control unit 6, which also receives inputs from various sensors 7 in the usual way. During use, the heating element 5 is submerged in water 8 below a rack 9 on which are placed articles 10 to be sterilized.

The open end 3 of the chamber 1 is closed, during a treatment cycle, by a door 11 of conventional construction. The door 11 is hinged about a vertical axis along one side and is locked by a conventional locking mechanism. The rear face 12 of the door 11, when closed, abuts against a seal 20 of annular shape extending around the flange 4 and supported on the inside of the lip 14. The seal 20 is made of a resilient, heat-resistant material, such as rubber, and is V-shape in section with two ribs 21 and 22 extending generally radially inwardly of the seal. The two ribs 21 and 22 diverge outwardly away from one another at an angle of about 90 degrees when uncompressed, the inner rib 22 being shorter than the outer rib 21. The outer rib 21 bears against the inner face 12 of the door 11, whereas the inner rib 22 bears against a parallel, forward face 40 of the flange 4. The flange 4 has a drain aperture 41 extending through it and opening just above the lower part of the annular seal 20 and just above the point where the inner rib 22 of the seal contacts the flange. The aperture 41 slopes down from the forward face 40 of the flange 4 to the rear face 42 and is coupled at the rear with a pipe 43. The pipe 43 and aperture 41 provide a passage extending to a reservoir 44 (which may be the main reservoir by which water is supplied to the chamber) via a solenoid valve 45. The valve 45 is controlled by electrical signals from the control unit 6.

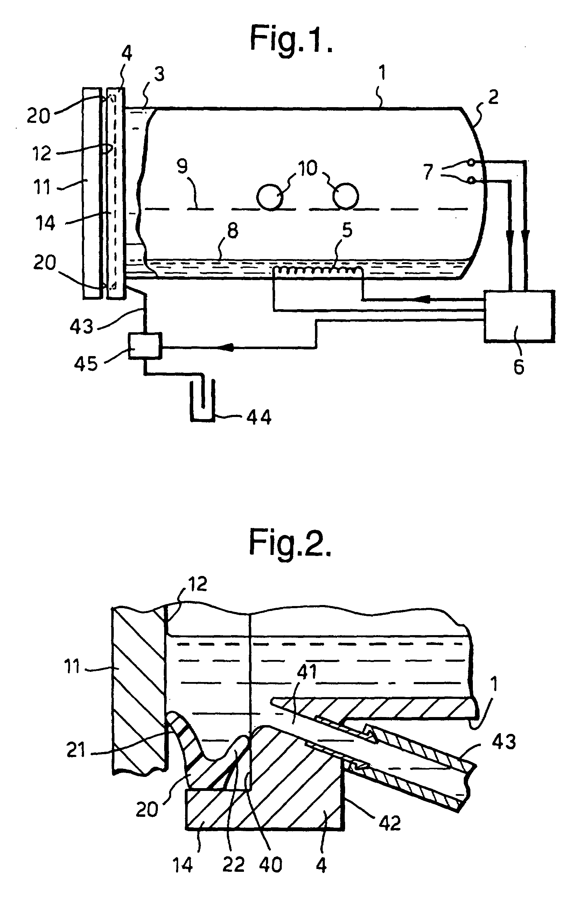

To use the autoclave, the articles 10 to be sterilized are placed on the rack 9 and the door 11 is closed so that it compresses the ribs 21 and 22 of the seal 20 slightly towards one another. The ribs 21 and 22 apply a resilient sealing force to the rear face 12 of the door 11 and to the forward face 40 of the flange 4. The autoclave then admits water from the reservoir 44 to the chamber 1 via the aperture 41 and performs a conventional sterilization treatment cycle. During this, the control unit 6 holds the valve 45 closed so that neither water nor pressure can escape through the drain aperture 41. As pressure rises in the chamber 1, this applies an outward pressure on the two ribs 21 and 22 of the seal 20, so as to increase the sealing pressure. During the sterilization cycle, water lies in the space above the seal 20, between the flange 4 and the inside face 12 of the door 11, as shown in Figure 2. At the end of the sterilization cycle, the control unit 6 opens the valve 45 and hence the passage provided through the aperture 41 and pipe 43 to the reservoir 44. Because the valve 45 is opened when pressure in the chamber 1 is still higher than atmosphere, the elevated pressure will force water in the chamber 1 out through the aperture 41 and into the reservoir 44. The only water remaining on the seal 20 will be that lying between the ribs 21 and 22 up to the level of the drain aperture 41. Because the outer rib 21 extends above the lower edge of the drain aperture 41, the collected water will not leak out when the door 11 is opened.

This arrangement, therefore, avoids the escape of water from the autoclave when the door is opened and thereby reduces the risk of damage or injury caused by contact with leaked water, which could still be hot.

## Claims

1. An autoclave having a chamber (1), a door (11) closing an opening to the chamber and an annular seal (20), extending around a flange (4) of the opening of the chamber, between the chamber and the door, **characterised in that** the flange (4) has a passage (41,43) opening just above the lower part of the annular seal, and the autoclave includes a valve (45) that prevents flow of liquid through the passage (41,43) from the chamber (1) during a treatment cycle and allows liquid collecting above the seal (20) to flow away before opening the door (11) after a treatment cycle.

2. An autoclave according to claim 1, wherein the seal (20) has an outer and inner resilient rib (21 and 22), and the outer rib (21) bears against an inner surface of the door (11) and the inner rib (22) bears against an outer surface (40) of the chamber.

3. An autoclave according to claim 2, wherein the two ribs (21 and 22) diverge away from one another and form a V shape in section.

4. An autoclave according to claim 2 or 3, wherein the outer rib (21) is longer than the inner rib (22).

5. An autoclave according to claim 4, wherein the outer rib (21) at the lower part of the seal (20) projects above the lower edge of the opening (41) of the passage (41,43).

6. An autoclave according to any one of the preceding claims, wherein the seal (20) is of a rubber.

7. An autoclave according to any one of the preceding claims, wherein the valve is a solenoid valve (45).

8. An autoclave according to any one of the preceding claims, wherein the passage (41,43) connects with a reservoir (44) by which liquid is supplied to the chamber (1) before a treatment cycle.

9. An autoclave according to any one of the preceding claims, wherein the flange has a forwardly-projecting lip (14), and the seal (20) is located on the lip (14).

## Patentansprüche

1. Autoklave, mit einer Kammer (1), einer Tür (11), die eine Öffnung der Kammer verschließt, und einer ringförmigen Dichtung (20), die sich um einen Flansch (4) der Öffnung der Kammer zwischen der Kammer und der Tür erstreckt, **dadurch gekennzeichnet, dass** der Flansch (4) einen Durchgang (41, 43) hat, der sich unmittelbar über dem unteren Teil der ringförmigen Dichtung öffnet, wobei der Autoklave ein Ventil (45) enthält, das den Fluss einer Flüssigkeit durch den Durchgang (41, 43) von der Kammer (1) während eines Behandlungszyklus verhindert und es Flüssigkeit ermöglicht, die sich oberhalb der Dichtung (20) ansammelt, abzufließen, bevor die Tür (11) nach einem Behandlungszyklus geöffnet wird.

2. Autoklave nach Anspruch 1, wobei die Dichtung (20) eine äußere und eine innere elastische Rippe (21 und 22) hat, wobei die äußere Rippe (21) gegen einer inneren Oberfläche der Tür (11) gelagert ist und die innere Rippe (22) gegenüber einer äußeren Oberfläche (40) der Kammer gelagert ist.

3. Autoklave gemäß Anspruch 2, wobei die zwei Rippen (21 und 22) zueinander auseinandergehen und im Schnitt eine V-Form bilden.

4. Autoklave gemäß Anspruch 2 oder 3, wobei die äußere Rippe (21) länger als die innere Rippe (22) ist.

5. Autoklave gemäß Anspruch 4, wobei die äußere Rippe bzw. Lippe (21) an dem unteren Teil der Dichtung (20) über die untere Kante der Öffnung (41) des Durchgangs (41, 43) erstreckt ist.

6. Autoklave nach einem der voranstehenden Ansprüche, wobei die Dichtung (20) aus Gummi ist.

7. Autoklave gemäß irgendeinem der voranstehenden Ansprüche, wobei das Ventil ein Solenoidventil (45) ist.

8. Autoklave gemäß einem der voranstehenden Ansprüche, wobei der Durchgang (41, 43) mit einem Reservoir (44) verbunden ist, durch das der Kammer (1) vor einem Behandlungszyklus Flüssigkeit zugeführt wird.

9. Autoklave gemäß irgendeinem der voranstehenden Ansprüche, wobei der Flansch eine nach vorne erstreckte Lippe (14) hat und die Dichtung (20) auf der Lippe (14) platziert ist.

## Revendications

1. Autoclave ayant une chambre (1), une porte (11) fermant une ouverture menant à la chambre et un joint annulaire (20) d'étanchéité, s'étendant le long d'un rebord (4) de l'ouverture de la chambre, entre la chambre et la porte, **caractérisé en ce que** le rebord (4) présente un passage (41, 43) s'ouvrant juste au-dessus de la partie inférieure du joint annulaire d'étanchéité, et l'autoclave comprend une vanne (45) qui empêche l'écoulement de liquide dans le passage (41, 43) depuis la chambre (1) pendant un cycle de traitement et qui permet à du liquide recueilli au-dessus du joint d'étanchéité (20) de s'éloigner en s'écoulant avant l'ouverture de la porte (11) après un cycle de traitement.

2. Autoclave selon la revendication 1, dans lequel le joint d'étanchéité (20) comporte des nervures élastiques extérieure et intérieure (21 et 22), et la nervure extérieure (21) porte contre une surface intérieure de la porte (11) et la nervure intérieure (22) porte contre une surface extérieure (40) de la chambre.

3. Autoclave selon la revendication 2, dans lequel les deux nervures (21 et 22) divergent l'une de l'autre et forment une configuration en V en section.

4. Autoclave selon la revendication 2 ou 3, dans lequel la nervure extérieure (21) est plus longue que la nervure intérieure (22).

5. Autoclave selon la revendication 4, dans lequel la nervure extérieure (21) à la partie inférieure du joint d'étanchéité (20) fait saillie au-dessus du bord inférieur de l'ouverture (41) du passage (41, 43).

6. Autoclave selon l'une quelconque des revendications précédentes, dans lequel le joint d'étanchéité (20) est en caoutchouc.

7. Autoclave selon l'une quelconque des revendications précédentes, dans lequel la vanne est une électrovanne (45).

8. Autoclave selon l'une quelconque des revendications précédentes, dans lequel le passage (41, 43) est raccordé à un réservoir (44) par lequel du liquide est amené à la chambre (1) avant un cycle de traitement.

9. Autoclave selon l'une quelconque des revendications précédentes, dans lequel le rebord comporte une lèvre (14) faisant saillie vers l'avant et le joint d'étanchéité (20) est placé sur la lèvre (14).
